# EUROPÄISCHE PATENTSCHRIFT

(11) **EP 0 432 354 B1**
(45) Veröffentlichungstag und Bekanntmachung des Hinweises auf die Patenterteilung: **29.06.1994**
(21) Anmeldenummer: 90113177.1
(22) Anmeldetag: 10.07.1990
(51) Int. Cl.: A61K 7/50

(54) **Badezusatz und dessen Verwendung**
Bath additive and its use
Additif pour bains et son utilisation

(30) Priorität: 08.12.1989 DE 3940704
(43) Veröffentlichungstag der Anmeldung: 19.06.1991
(73) Patentinhaber: Apotheker Hans Starke, Vertrieb chemisch-pharmazeutischer Präparate GmbH, D-82301 Starnberg (DE)
(72) Erfinder: Kemp, Helmut, D-6624 Grossrosseln 4 (DE)
(74) Vertreter: Vogeser, Werner, Dipl.-Ing.

(56) Entgegenhaltungen:
- DE-A- 2 231 887
- FR-A- 2 208 643
- FR-A- 2 432 311
- GB-A- 2 182 943
- GB-A- 2 200 636
- Rote Liste 87, Seite 25ff

## Beschreibung

Die Erfindung betrifft einen Badezusatz auf der Basis pflanzlicher und/oder tierischer Rohstoffe, sowie die Verwendung des Badezusatzes zur Behandlung von Hautkrankheiten.

Hautkrankheiten unter denen eine Vielzahl von Menschen leiden, lassen sich prinzipiell nach ihrer Ursache in durch Bakterien, Protozoen, Viren, Parasiten oder Pilze verursachte entzündliche Hautkrankheiten, z.B. Syphillis, Gürtelrose, Krätze, allergische und autoimmunbedingte Hautkrankheiten, z. B. endogene Ekzeme, und in ihrer Ursache nach unbekannte Hautkrankheiten, z.B. Schuppenflechte, unterteilen. Gegen mykotische, bakterielle und virale Hautkrankheiten werden in der Regel antimikrobiell wirkende Salben eingesetzt, die jedoch eine hautreizende Wirkung zeigen und den Säuremantel der Haut negativ so beeinflussen können, daß ein erneuter Befall durch Pilze und Bakterien eintritt. Bei Behandlung der Schuppenflechte kommen meistens cortisonhaltige Salben oder eine kombinierte Behandlung der Einnahme eines Fotosensibilisators, z.B. eines Retinoids, und nachfolgender UV-A-Bestrahlung zur Anwendung. Damit kann jedoch zum einen der Mineralstoffhaushalt gestört und die Haut schlaff werden, zum anderen kann eine sehr starke Hautschädigung auftreten. Die Behandlung mit diesen Mitteln ist jedoch meist nur kurzfristig von Erfolg und ist von sehr starken Nebenwirkungen begleitet.

Das wachsende Bedenken gegen die Verwendung und die z.T. nicht vorhersehbaren Nebenwirkungen dieser Arzneimittel haben daher bei den von diesen Hautkrankheiten befallenen Menschen und auch bei den Herstellern dieser Arzneimittel dazu geführt, nach schonenden Mitteln auf pflanzlicher oder tierischer Basis zur Behandlung dieser Hautkrankheiten zu suchen.

Aus der FR-A-2 208 643 ist eine Antischuppenlotion bekannt, die pflanzliche Extrakte, Detergenzien und eine wäßrige alkoholische Lösung enthält.

Die GB-A-2 182 943 beschreibt ein einen Pflanzenextrakt enthaltendes Haarshampoo, welches zur Behandlung des menschlichen Körpers bei Läusebefall oder bei anderen Ektoparasiten geeignet ist und auf einem Knoblauch- und/oder Petersilieextrakt basiert.

Die DE-A-2 231 887 beschreibt ein Badeflocken-Spray aus verschiedenen oberflächenaktiven Substanzen, pflanzlichen Ölen sowie pflanzlichen und tierischen Extrakten und verschiedenen Alkoholen.

Aus der GB-A-2 200 636 ist eine kosmetische Zusammensetzung bekannt, die ein schäumendes oberflächenaktives Mittel enthält. Die bekannte Zusammensetzung kann ein anionisches, nichtionisches, amphoteres, zwitterionisches oder kationisches oberflächenaktives Mittel, Wasser und/oder einen Alkohol enthalten.

Badezusätze mit Heilwirkung sind in Rote Liste 87 beschrieben.

Aufgabe der Erfindung ist es daher, einen Badezusatz auf Basis pflanzlicher und/oder tierischer Rohstoffe und dessen Verwendung in medizinischen Bädern bereitzustellen, mit dem ohne schädigende Einflüsse auf die Haut die Folgen von Hautkrankheiten weitgehend gelindert oder sogar vollständig beseitigt werden können.

Diese Aufgabe wird erfindungsgemäß durch einen Badezusatz auf der Basis pflanzlicher und/oder tierischer Rohstoffe gemäß den Merkmalen der Patentansprüche 1, 4 und 10 gelöst. Bevorzugte Weiterführungen der Erfindung sind in den Unteransprüchen angegeben.

Gemäß einer Ausführungsform der Erfindung enthält der Badezusatz zumindest eine wässrige Lösung eines Gemisches amphoterer und/oder anionischer und/oder nichtionischer Tensiden (Tensidgemisch) auf der Basis pflanzlicher und/oder tierischer Fette und Öle und eine alkoholische Lösung auf der Basis einer oder mehrerer Heilpflanzen. Unter wässriger Lösung ist dabei im Sinne der Erfindung das Gemisch aus gelösten bzw. in Lösung sich befindlichen Tensiden und eingesetztem Wasser zu verstehen. Als pflanzliche und/oder tierische Fette und Öle können erfindungsgemäß solche aus Talgdrüsen von Schafen,Kokosnuß, Ricinus, Sojabohne oder dgl. eingesetzt werden. Durch Einsatz dieser Lösungen in dem Badezusatz ist sowohl eine antibiotische Wirkung als auch eine Linderung entzündlicher Prozesse möglich. Wegen des Fehlens schädigender chemischer Substanzen, z.B. Cortisone, treten keine auf die Haut oder den Mineralstoffhaushalt negativ einwirkenden Begleiterscheinungen auf.

Der aus den Rohstoffen erhaltene Badezusatz der vorliegenden Erfindung enthält 85 bis 95 Gew.-% der wässrigen Lösung auf der Basis der pflanzlichen und/oder tierischen Öle und Fette, 5 bis 15 Gew.-% der alkoholischen Lösung auf der Basis der Heilpflanzen und bis zu 1,5 Gew.-% vorzugsweise 0,5 bis 1,3 Gew.-% anorganische Salze. Mit dieser Zusammensetzung treten zu dem synergistischen Effekt der grenzflächenaktiven Substanzen die hautfreundliche Wirkung der Heilpflanzen sowie die gewünschte Einstellung der Viskosität und die für die Haut günstige Einstellung des pH-Wertes im neutralen bis schwach sauren Bereich.

Vorzugsweise ist das als Verdicker bzw. als Viskositätsregler eingesetzte anorganische Salz Natriumchlorid und der pH-Wert einer 1%igen Lösung des Badezusatzes 6,5 - 7,5, vorzugsweise 7,3.

Es hat sich als vorteilhaft erwiesen, daß die wässrige Lösung Feststoffe in einer Menge von 18 bis 24 Gew.-%, 4 bis 8 Gew.-% eines Lösungsmittels bzw. Lösungsvermittlers sowie 70 bis 75 Gew.-% Wasser, bezogen auf das Gesamtgewicht der wässrigen Lösung, enthält. Als das die Ausflockung der Feststoffe in Wasser verhindernde Lösungsmittel wird ein niederer Alkohol, vorzugsweise Ethanol oder Isopropylalkohol in einer Reinheit von mindestens 90 Vol.-%, eingesetzt, wobei die mit anderen organischen Lösungsmitteln verbundenen gesundheitlichen Bedenken ausgeschaltet werden. Es hat sich dabei herausgestellt, daß bei einer in dem erfindungsgemäßen Bereich liegenden Zusammensetzung der wässrigen Lösung die Wirkung auf die befallenen Hautbereiche am größten ist.

Zum Erreichen der beabsichtigten Wirkung können die Feststoffe der wässrigen Lösung auf Basis eines pflanzlichen und/oder tierischen Fettes oder Öles 2 bis 3 Gew.-%, vorzugsweise 2,5 Gew.-%, amphotere Tenside, 11,1 bis 12,5 Gew.-%, vorzugsweise 12,0-Gew.-% anionische Tenside und 4,8 bis 5,9 Gew.-%, vorzugsweise 5,5 Gew.-%, nichtionische Tenside enthalten. Mit dieser Zusammensetzung sowie einem Gehalt an 73 Gew.-% Wasser und 5 Gew.-% Lösungsvermittler ist die wässrige Lösung des erfindungsgemäßen Badezusatzes besonders vorteilhaft.

Gemäß einer weiteren Ausführungsform der Erfindung enthält der Badezusatz 91 bis 97 Gew.-% der wässrigen Lösung auf der Basis des pflanzlichen und/oder tierischen Fettes und Öles, 0,01 bis 8 Gew.-% der alkoholischen Lösung auf der Basis der Heilpflanzen und bis zu 4 Gew.-% vorzugsweise 2,7 bis 3,8 Gew.-% anorganische Salze. Die gleichen pflanzlichen und/oder tierischen Fette und Öle wie bei der ersten Ausführungsform kommen dabei zum Einsatz, wobei der Badezusatz im wesentlichen die gleiche Wirkung zeigt. Das als Verdicker bzw. als Viskositätsregler eingesetzte anorganische Salz ist vorzugsweise Natriumchlorid und der pH-Wert einer 1%-igen Lösung des Badezusatzes beträgt von 6,8 bis 7,4.

Die wässrige Lösung des Badezusatzes gemäß vorstehender Ausführungsform enthält 3 bis 7 Gew.-%, vorzugsweise 4,8 bis 6,5 Gew.-% amphotere Tenside, 40 bis 55 Gew.-% vorzugsweise 43 bis 49 Gew.-%, anionische Tenside, 1 bis 8 Gew.-%, vorzugsweise 3,8 bis 6,5 Gew.-% nichtionische Tenside, 30 bis 40 Gew.-%, vorzugsweise 33,5 bis 38,5 Gew.-% Wasser sowie 1 bis 5 Gew.-%, vorzugsweise 2,5 bis 4,2 Gew.-% eines Lösungsmittels bzw. Lösungsvermittlers, wobei sich die Gew.-%-Angaben auf das Gesamtgewicht des Badezusatzes beziehen. Der die Ausflockung der Tenside im Wasser verhindernde Lösungsvermittler ist wie bei der ersten erfindungsgemäßen Ausführungsform ein niederer Alkohol, vorzugsweise Ethanol oder Isopropylalkohol einer Reinheit von mindestens 90 Vol-%.

Erfindungsgemäß werden als amphotere Tenside ein Aminocarboxylat oder eine Aminoverbindung mit Betainstruktur, als anionisches Tensid ein Salz eines Alkylpolyglykolethersulfats und als nichtionisches Tensid ein Fettalkoholpolyglykolether auf der Basis von Kokosöl eingesetzt. Diese Tenside werden in an sich bekannter Weise aus den in den Samen der Kokospalme enthaltenen Kokosfettsäuren gewonnen und entsprechend umgesetzt und sind in der vorstehend genannten Menge von im wesentlichen physiologischer und medizinischer Unbedenklichkeit.

Das aus dem Kokosöl gewonnene amphotere Tensid ist gemäß der Erfindung ein mit Alkyl substituiertes Ammoniumbetain, ein Dicarboxyldiamin mit Betainstruktur oder ein Fettsäureamidderivat mit Betainstruktur, das anionische Tensid das Natriumsalz von sulfatierten C₁₂- bis C₁₄-Fettalkoholen mit einer Ethoxylierungszahl (EO-Zahl) von 2 oder 3 und ein C₁₂- bis C₁₄ Fettalkoholpolyglykolether mit einer EO-Zahl von 4,5 oder 6. Als amphotere Tenside seien hier handelsübliches Kokosalkyldimethylammoniumbetain, Lauryldimethylcarboxymethylammoniumbetain und Dimethylcarboxymethyl-Kokosfettsäurepropylamidoammoniumbetain (Rewoteric ® AM B 13 der Firma REWO) genannt, die, wie auch die vorstehend genannten handelsüblichen anionischen und nichtionischen Tenside, in der pharmazeutischen und kosmetischen Industrie häufig zur Anwendung kommen und physiologisch unbedenklich sind. Als für die Erfindung besonders geeignet gezeigt haben sich als anionisches Tensid Natriumlaurylethersulfat (Rewopol ®NL 3 der Firma REWO) sowie als nichtionisches Tensid Laurylalkoholpolyglykolether (Rewopal ® LA 6 der Firma REWO).

Zur Linderung der durch die Hautkrankheiten hervorgerufenen entzündlichen Prozesse sowie zur Neutralisation des eventuell auftretenden Rohstoffeigengeruchs der aus Fetten oder Ölen stammenden Tenside werden erfindungsgemäß durch Glykolextraktion in an sich bekannter Weise erhaltene pflanzliche Extrakte von Schafgarbe, Haferstroh und/oder Kamille eingesetzt, deren desinfizierende, schmerzlindernde und abschwellende Wirkung ihrer Wirkstoffe, z.B. ätherische Öle, Alkaloide, Aminosäuren und Glykoside, aus der Pflanzenheilkunde bekannt sind. Als für die vorliegende Erfindung vorteilhaft ist der Einsatz von Schafgarben-, Haferstroh- und Kamillen-Extrakten in einer Menge von 2 bis 8 Gew.-% eines jeden Extraktes, bezogen auf das Gesamtgewicht des Badezusatzes, gemäß der ersten Ausführungsform bzw in einer Menge von 0,01 bis 8 Gew.-% eines jeden Extraktes, bezogen auf das Gesamtgewicht des Badezusatzes, gemäß der zweiten Ausführungsform, wobei die Menge im wesentlichen von der Konzentration des Heilpflanzenextrakts in der alkoholischen Lösung in beiden Ausführungsformen abhängt. Im Rahmen der vorliegenden Erfindung liegt selbstverständlich auch der Einsatz eines pflanzlichen Öls anstelle einer alkoholischen Lösung der pflanzlichen Extrakte.

Der Badezusatz auf der Basis pflanzlicher und/oder tierischer Rohstoffe gemäß vorliegender Erfindung findet als Mittel zur Behandlung mikrobiell bedingter sowie chronisch endogener Hautkrankheiten, vorzugsweise zur Behandlung der Schuppenflechte in medizinischen Bädern Verwendung. Der Badezusatz kann alleine oder unterstützend zur medizinischen Behandlung der Hautkrankheiten eingesetzt werden. Eine Mischung aus 90 Gew.-% der wässrigen Lösung und 10 Gew.-% der Heilpflanzenextrakte gemäß der ersten Ausführungsform, die in einem Verhältnis Badezusatz : Wasser von 1 : 400 bis 1 : 1000 angewendet wird bzw. ca.97 bis 99.9 Gew.-% einer aus ca. 96 bis 98 Gew.-% wässriger Lösung und ca. 1 bis 4 Gew.-% anorganische Salze bestehender Lösung sowie 0,02 bis 2 Gew.-% Heilpflanzenextrakt gemäß der zweiten Ausführungsform, die in einem Verhältnis Badezusatz: Wasser von 1 : 250 bis 1 : 800 angewendet wird, hat sich dabei als besonders vielversprechend erwiesen. Ein mehrmaliges, 25 bis 30 minütiges Sitzen oder Liegen in dem vorstehend genannten Badewasser führt dabei zu einer Erfolgsquote von 90 bis 95% bei Schuppenflechte.

Die Erfindung wird nachfolgend anhand eines Beispiels näher erläutert.

In einem Behälter entsprechender Größe wird zuerst eine erste Lösung aus einem handelsüblich erhältlichen Kamillenextrakt in der in der nachfolgenden Tabelle angegebenen Menge mit einer Teilmenge eines Laurylalkoholpolyglykolethers (EO-Zahl 6) in einem Verhältnis Kamillenextrakt : Laurylalkoholpolyglykolether von 1:50 bis 1:100 hergestellt und während 5 bis 10 Minuten gerührt. Anschließend wird in einem weiteren Behälter das zum Einstellen der Viskosität benötigte Natriumchlorid in entweder mit von mikrobiellen Verunreinigungen befreitem Wasser ("gereinigtes Wasser") oder Leitungswasser einer Temperatur von 60 bis 80°C in der in der Tabelle angegebenen Mengen zur Herstellung einer zweiten Lösung vollständig gelöst. In einen dritten Behälter werden dann nacheinander die erste Lösung, die Restmenge des Laurylalkoholpolyglykolethers, die gemäß der Tabelle angegebenen Mengen an Isopropylalkohol, handelsüblich erhältlichem Dimethylcarboxymethyl-Kokosfettsäurepropylamidoammoniumbetain sowie Natriumlaurylethersulfat (EO-Zahl 3) und die zweite Lösung eingebracht und mit einem Turborührer oder dgl. einer Leistung von 2 KW während ca. 10 min gerührt. Die so erhaltene Lösung wird dann während 18 bis 24 h stehengelassen, anschließend noch einmal mit dem Turborührer während 5 min durchmischt und nach völliger Schaumfreiheit der erfindungsgemäße Badezusatz abgepackt.

**Tabelle**

| | |
|---|---|
| Kamillenextrakt | 0,03 g |
| Laurylalkoholpolyglykolether (Rewopal ® LA 6) | 5,50 g |
| Isopropylalkohol | 3,00 g |
| Dimethylcarboxymethyl-Kokosfettsäurepropylamidoammoniumbetain (Rewoteric ® AM B 13) | 6,00 g |
| Natriumlaurylethersulfat (Rewopol ® NL 3) | 45,00 g |
| NaCl | 3,5 g |
| Wasser ("gereinigtes Wasser") | 36,97 g |

## Patentansprüche

1. Badezusatz auf der Basis pflanzlicher und/oder tierischer Rohstoffe, enthaltend eine wäßrige Lösung eines Gemisches amphoterer und/oder anionischer und/oder nichtionischer Tenside auf der Basis pflanzlicher und/oder tierischer Fette und Öle und eine alkoholische Lösung auf der Basis einer oder mehrerer Heilpflanzen,
**dadurch gekennzeichnet,** daß
der Badezusatz 85 bis 95 Gew.-% der wäßrigen Lösung eines Gemisches amphoterer, anionischer und nichtionischer Tenside auf der Basis des pflanzlichen und/oder tierischen Fettes und Öles, 5 bis 15 Gew.-% der alkoholischen Lösung auf der Basis der Heilpflanzen und bis zu 1,5 Gew.-% anorganische Salze enthält.

2. Badezusatz nach Anspruch 1,
**dadurch gekennzeichnet,** daß
die wäßrige Lösung Feststoffe in einer Menge von 18 bis 24 Gew.-%, 4 bis 8 Gew.-% eines Lösungsmittels bzw. Lösungsvermittlers sowie 70 bis 75 Gew.-% Wasser, bezogen auf das Gesamtgewicht der wäßrigen Lösung, enthält.

3. Badezusatz nach Anspruch 2,
**dadurch gekennzeichnet,** daß
die Feststoffe der wäßrigen Lösung von 2 bis 3 Gew.-% amphoterer Tenside, 11,3 bis 12,5 Gew.-% anionischer und 4,8 bis 5,9 Gew.-% nichtionischer Tenside sind.

4. Badezusatz auf der Basis pflanzlicher und/oder tierischer Rohstoffe, enthaltend eine wäßrige Lösung eines Gemisches amphoterer und/oder anionischer und/oder nichtionischer Tenside auf der Basis pflanzlicher und/oder tierischer Fette und Öle und eine alkoholische Lösung auf der Basis einer oder mehrerer Heilpflanzen,
**dadurch gekennzeichnet,** daß
der Badezusatz 91 bis 97 Gew.-% der wäßrigen Lösung eines Gemisches amphoterer, anionischer und nichtionischer Tenside auf der Basis des pflanzlichen und/oder tierischen Fettes und 0,01 bis 8 Gew.-% einer alkoholischen Lösung und bis zu 4 Gew.-% anorganische Salze enthält.

5. Badezusatz nach Anspruch 4,
**dadurch gekennzeichnet,** daß
die wäßrige Lösung von 3 bis 7 Gew.-% amphotere Tenside, 40 bis 55 Gew.-% anionische Tenside, 1 bis 8 Gew.-% nichtionische Tenside, 30 bis 40 Gew.-% Wasser sowie 1 bis 5 Gew.-% eines Lösungsmittels bzw. Lösungsvermittlers, bezogen auf das Gesamtgewicht des Badezusatzes, enthält.

6. Badezusatz nach Anspruch 2 oder 5,
**dadurch gekennzeichnet,** daß
das Lösungsmittel bzw. der Lösungsvermittler ein niederer Alkohol ist.

7. Badezusatz nach einem der vorhergehenden Ansprüche,
**dadurch gekennzeichnet,** daß
das aus einem Fett oder Öl erhaltene amphotere Tenside ein Aminocarboxylat oder eine Aminoverbindung mit Betainstruktur, das anionische Tensid ein Salz eines Alkylpolyglykolethersulfats und das nichtionische Tensid ein Fettalkoholpolyglykolether auf der Basis von Kokosnußöl sind.

8. Badezusatz nach Anspruch 7,
**dadurch gekennzeichnet,** daß
die Aminoverbindung mit Betainstruktur ein mit Alkyl substituiertes Ammoniumbetain, das Salz des Alkylpolyglykolethersulfats das Natriumsalz von sulfatierten C₁₂-C₁₄-Fettalkoholen mit einer Ethoxylierungszahl von 2 oder 3 und der Fettalkoholpolyglykolether ein C₁₂-C₁₄-Fettalkoholpolyglykolether mit einer Ethoxylierungszahl von 4,5 oder 6 sind.

9. Badezusatz nach einem der vorhergehenden Ansprüche,
**dadurch gekennzeichnet,** daß
der Badezusatz in an sich bekannter Weise erhaltene Heilpflanzenextrakte von Schafgarbe, Haferstroh und/oder Kamille enthält.

10. Verwendung eines Badezusatzes nach einem der Ansprüche 1 bis 9 zur Herstellung eines äußerlich anwendbaren Mittels zur Behandlung mikrobiell bedingter sowie chronisch endogener Hautkrankheiten.

## Claims

1. Bath additive based on vegetable and/or animal raw materials, containing an aqueous solution of a mixture of amphoteric and/or anionic and/or nonionic surfactants based on vegetable and/or animal fats and oils and an alcoholic solution based on one or more medicinal herbs,
characterised in that the bath additive contains from 85 to 95 wt-% of the aqueous solution of a mixture of amphoteric, anionic and nonionic surfactants based on the vegetable and/or animal fat and oil, from 5 to 15 wt-% of the alcoholic solution based on the medicinal herbs and up to 1.5 wt-% inorganic salts.

2. Bath additive according to Claim 1, characterised in that the aqueous solution contains solids in a quantity of from 18 to 24 wt-%, from 4 to 8 wt-% of a solvent or of a solubility promoter and from 70 to 75 wt-% water, calculated on total weight of the aqueous solution.

3. Bath additive according to Claim 2, characterised in that the solids of the aqueous solution are from 2 to 3 wt-% amphoteric surfactants, from 11.3 to 12.5 wt-% anionic surfactants and from 4.8 to 5.9 wt-% nonionic surfactants.

4. Bath additive based on vegetable and/or animal raw materials, containing an aqueous solution of a mixture of amphoteric and/or anionic and/or nonionic surfactants based on vegetable and/or animal fats and oils and an alcoholic solution based on one or more medicinal herbs, characterised in that the bath additive contains from 91 to 97 wt-% of the aqueous solution of a mixture of amphoteric, anionic and nonionic surfactants based on the vegetable and/or animal fat and from 0.01 to 8 wt-% of an alcoholic solution and up to 4 wt-% inorganic salts.

5. Bath additive according to Claim 4, characterised in that the aqueous solution contains from 3 to 7 wt-% amphoteric surfactants, from 40 to 55 wt-% anionic surfactants, from 1 to 8 wt-% nonionic surfactants, from 30 to 40 wt-% water and from 1 to 5 wt-% of a solvent or of a solubility promoter, calculated on total weight of the bath additive.

6. Bath additive according to Claim 2 or 5, characterised in that the solvent or solubility promoter is a low alcohol.

7. Bath additive according to one of the preceding claims, characterised in that the amphoteric surfactant obtained from a fat or oil is an aminocarboxylate or an amino compound having betaine structure, the anionic surfactant is a salt of an alkylpolyglycol ether sulphate and the nonionic surfactant is a fatty alcohol polyglycol ether based on coconut oil.

8. Bath additive according to Claim 7, characterised in that the amino compound having betaine structure is an alkyl-substituted ammonium betaine, the salt of the alkylpolyglycol ether sulphate is the sodium salt of sulphated C₁₂-C₁₄ fatty alcohols having an ethoxylation number of 2 or 3, and the fatty alcohol polyglycol ether is a C₁₂-C₁₄ fatty alcohol polyglycol ether having an ethoxylation number of 4, 5 or 6.

9. Bath additive according to one of the preceding claims, characterised in that the bath additive contains medicinal herb extracts of milfoil, oat straw and/or camomile which are obtained in a manner known per se.

10. Use of a bath additive according to one of Claims 1 to 9 for the preparation of an agent which may be applied externally for the treatment of skin diseases due to microorganisms and to chronically endogenous causes.

## Revendications

1. Complément de bain à base de matières premières végétales et/ou animales contenant une solution aqueuse d'un mélange d'agents tensio-actifs amphotères et/ou anioniques et/ou non ioniques à base de graisses et d'huiles végétales et/ou animales et une solution alcoolique à base d'une ou plusieurs plantes curatives, caractérisé en ce que le complément de bain contient 85 à 95 % en poids de la solution aqueuse d'un mélange d'agents tensio-actifs amphotères, anioniques et nonioniques à base de graisses et d'huiles végétales et/ou animales, 5 à 15 % en poids de la solution alcoolique à base de plantes curatives et jusqu'à 1,5 % en poids de sels minéraux.

2. Complément de bain selon la revendication 1, caractérisé en ce que la solution aqueuse contient des matières solides en quantité de 18 à 24 % en poids, 4 à 8 % en poids d'un solvant ou agent de solubilisation ainsi que 70 à 75 % en poids d'eau, sur base du poids total de la solution aqueuse.

3. Complément de bain selon la revendication 2, caractérisé en ce les substances solides de la solution aqueuse sont constituées de 2 à 3 % en poids d'agents tensio-actifs amphotères, de 11,3 à 12,5 % en poids d'agents anioniques et de 4,8 à 5,9 % en poids d'agents tensio-actifs non ioniques.

4. Complément de bain à base de matières premières végétales et/ou animales, contenant une solution aqueuse d'un mélange d'agents tensio-actifs amphotères et/ou anioniques et/ou non ioniques à base de graisses et d'huiles végétales et/ou animales et une solution alcoolique à base d'une ou plusieurs plantes curatives, caractérisé en ce que le complément de bain contient 91 à 97 % en poids de la solution aqueuse d'un mélange d'agents tensio-actifs amphotères, anioniques et nonioniques à base de graisses végétales et/ou animales et 0,01 à 8 % en poids d'une solution alcoolique et jusqu'à 4 % en poids de sels minéraux.

5. Complément de bain selon la revendication 4, caractérisé en ce que la solution aqueuse contient 3 à 7 % en poids d'agents tensio-actifs amphotères, 40 à 55 % en poids d'agents tensio-actifs anioniques, 1 à 8 % en poids d'agents tensio-actifs non ioniques, 30 à 40 % en poids d'eau ainsi que 1 à 5 % en poids d'un solvant ou agent de solubilisation par rapport au poids total du complément de bain.

6. Complément de bain selon la revendication 2 ou 5, caractérisé en ce que le solvant ou agent de solubilisation est un alcool inférieur.

7. Complément de bain selon l'une quelconque des revendications précédentes, caractérisé en ce que l'agent tensio-actif amphotère obtenu à partir d'une graisse ou d'une huile est un aminocarboxylate ou un composé aminé à structure de bétaïne, l'agent tensio-actif anionique est un sel d'un éther sulfate d'alkylpolyglycol et l'agent tensio-actif non ionique est un polyglycoléther d'alcool gras à base d'huile de noix de coco.

8. Complément de bain selon la revendication 7, caractérisé en ce que le composé aminé à structure de bétaïne est une bétaïne d'ammonium substituée par un groupe alkyle, le sel d'éthersulfate d'alkylpolyglycol est le sel de sodium d'alcools gras en C₁₂-C₁₄ sulfatés ayant un indice d'éthoxylation de 2 ou 3 et le polyglycoléther d'alcool gras est un polyglycoléther d'alcool gras en C₁₂-C₁₄ avec un indice d'éthoxylation de 4, 5 ou 6.

9. Complément de bain selon l'une quelconque des revendications précédentes, caractérisé en ce que le complément de bain contient des extraits de plantes curatives, obtenus de manière connue en soi, d'achilée, de paille d'avoine et/ou de camomille.

10. Utilisation d'un complément de bain selon l'une quelconque des revendications 1 à 9 pour la préparation d'un agent à usage externe pour le traitement d'affections de la peau endogènes d'origine microbienne et chronique.
